# EUROPEAN PATENT APPLICATION

(11) **EP 1 584 349 A1**
(43) Date of publication of application: **12.10.2005**
(21) Application number: 05252104.4
(22) Date of filing: 04.04.2005
(51) Int. Cl.: A61N 1/372, A61B 5/00

(54) **Hierarchical data storage and analysis system for implantable medical devices**

(30) Priority: 04.04.2004 US 819400
(71) Applicant: PACESETTER, INC., Sylmar, CA 91392-9221 (US)
(72) Inventor: Nirav, Dalal, North Hills, CA 91343 (US); Balakrishnan, Shankar, Valencia, CA 91355 (US)
(74) Representative: Rees, David Christopher

(57) **Abstract**

A hierarchical data storage and analysis system for implantable medical devices is described. In an implementation, a method includes analyzing data collected by an implantable medical device (IMD) by utilizing a set of analytical capabilities. A determination is made, from the analyzing, whether to communicate the data to a computing device having additional analytical capabilities and storage space that are not available at the IMD.

## Description

The present invention generally relates to implantable medical devices and ways to store and analyze data gathered by such devices.

Implantable medical devices (IMDs) are implanted within the body of a patient to monitor, regulate, and/or correct physiological conditions. Implantable medical devices include implantable cardiac stimulation devices (e.g., pacemakers, implantable defibrillators) that apply stimulation therapy to the heart, implantable cardiac monitors that monitor heart activity, implantable neurological devices that monitor and stimulate nerves, and other implantable devices used for diagnostic and/or therapeutic purposes, such as medication dosing devices.

Implantable medical devices typically include a control unit positioned within a casing that is implanted into the body. A set of leads provide an electrical interface between the encased control unit and the body. With improved processor and memory technologies, the control units have become increasingly more sophisticated, allowing them to monitor many types of physiological conditions and apply tailored therapies in response to those conditions.

Some implantable medical devices can be designed to communicate with, and/or be programmed by, systems that are external to the patient. For example, implantable cardiac devices are equipped with telemetry circuits that communicate with an external programmer via a proximally positioned electromagnetic wand. The wand contains a coil that forms a transformer coupling with the device's telemetry circuitry to transmit low frequency signals by varying coil impedance.

Early telemetry communication was unidirectional from the programmer to the implanted device. Passive telemetry allowed a treating physician to download instructions to the implanted device following implantation. Due to power and size constraints, early commercial versions of implanted devices were typically incapable of transmitting information back to the programmer.

As power capabilities improved, active telemetry became feasible, allowing synchronous bi-directional communication between the implanted device and the external system. One example of an active telemetry protocol is to utilize a half-duplex communication mode in which the programmer sends instructions in a predefined frame format and, following termination of this transmission, the implanted device returns data using the frame format. With active telemetry, the treating physician is able to not only program the implanted device, but also retrieve information from the implanted device to evaluate physiological activity and device performance. The treating physician may periodically want to review device performance or physiological data for predefined periods of time to ensure that the device is providing therapy in a desired manner. Consequently, current generation implantable cardiac therapy devices incorporate memories, and the processors periodically sample and record various performance parameter measurements in the memories. Data pertaining to a patient's condition can also be passed to and stored by the external system during communication sessions with the implanted device. This data can then be analyzed by the system.

One challenge that still persists, however, is how to efficiently and effectively store and analyze data captured by implantable medical devices.

According to one aspect of the invention, there is provided a system comprising: at least one implantable medical device (IMD) that collects data; and a plurality of computing devices that are communicatively coupled, one to another, characterized in that: each of the computing devices has capabilities to analyze the data; at least one of the computing devices is communicatively coupled to the IMD to obtain the data; and one or more of the computing devices is configured to determine whether to communicate the data to another one of the computing devices having analytical capabilities that are not included in the analytical capabilities of the one or more computing devices.

According to another aspect of the invention, there is provided a system comprising: a hierarchy of computing devices that are communicatively coupled, one to another, characterized in that: at least one of the computing devices is an implantable medical device (IMD); the hierarchy is defined by each respective amount of data that each of the computing devices is configured to store; and one or more of the computing devices is configured to determine whether to communicate the data to another one of the computing devices that is configured to store a different amount of data.

The invention also extends to a method comprising: analyzing data collected by an implantable medical device (IMD) by utilizing a set of analytical capabilities; and determining, from the analyzing, whether to communicate the data to a computing device having additional analytical capabilities that are not included in the set of analytical capabilities.

Preferably, the analyzing is performed by the IMD. Preferably, the analytical capabilities are selected from the group consisting of: processing capabilities; memory resources; network resources; patient diagnostic algorithms; device diagnostic algorithms; and any combination thereof.

The data may describe a patient or operation of the IMD.

Preferably, the analyzing and the determining are performed automatically and without user intervention, and the method further comprises notifying a designated user of a result of the analyzing. The method may further comprise: communicating the data to the computing device; analyzing the data by the computing device utilizing the additional analytical capabilities; and determining, from the analyzing by the computing device, whether to communicate the data to another computing device having analytical capabilities that are not included in the additional analytical capabilities.

The invention may also extend to a method comprising: collecting data by an implantable medical device (IMD); and managing distribution of the data to one or more of a plurality of computing devices based on respective capabilities of each of the computing devices to analyze the data.

Preferably, the managing further comprises communicating data from the IMD at predetermined intervals of time.

The managing may further comprise: analyzing the data by utilizing a set of analytical capabilities; determining, from the analyzing, whether to communicate the data to one of the computing devices having additional analytical capabilities that are not included in the set of analytical capabilities; and forming a communication for notifying a designated user of a result of the analyzing.

The method may further comprise: collecting data by another IMD; and aggregating the data from both IMDs, wherein the managing includes managing distribution of the aggregated data.

Preferably, in a network system including a plurality of computing devices, wherein at least one of the computing devices is an implantable medical device (IMD), at least one the computing devices is an intermediate computing device, and at least one of the computing devices is a endpoint device, wherein the IMD and the intermediate computing device each includes a communication agent and an analysis agent, the method comprises: analyzing data through execution of one of the analysis agents; and when the data is determined to be critical data, indicating the data is critical such that the data is communicated by the communication agent to the endpoint device without first being analyzed by another one of the analysis agents, otherwise, analyzing the data by the other one of the analysis agents before communication.

The analysis agent, when executed, may provide work flow management. The method may further comprise forming a communication for notifying a designated user of a result of the analyzing.

The invention may also extend to an implantable medical device comprising: memory for maintaining data; and one or more agents that are executable to: analyze the data; and communicate the data to an external device when the analyzing provides an indication for additional analysis which is not provided by the one or more agents.

Preferably, the one or more agents are further executable to form a communication for notifying a designated user when a result of the analyzing indicates a change in a patient's condition. The data may describe a patient's condition. The external device may be configured as a device selected from the group consisting of: a patient-wearable device; a networked programmer; and an offline programmer. The external device may be an intermediate computing device that is communicatively coupled to an endpoint device.

The invention also contemplates a network architecture for managing data output by an implantable medical device (IMD) through a plurality of computing devices, the network architecture comprising: a hierarchy of agents, wherein each of the agents is executable to analyze the data collected by the IMD; and at least one of the plurality of agents is operative to determine whether the agent has capabilities sufficient to analyze the data, and that is responsive determining that the agent does not have sufficient capabilities to cause the data to be communicated to another one of the agents that has capabilities sufficient to analyze the data.

One or more of the plurality of agents may be executable to form a communication, based on the analysis, for notifying a designated user of a result of the analysis. One or more of the plurality of agents may be executable to aggregate data from at least two IMDs. One or more of the plurality of agents may be executable to aggregate data from the IMD and an external medical device.

The invention also contemplates a network architecture for managing data output by a plurality of implantable medical devices (IMDs) to a plurality of computing devices, wherein at least one of the computing devices is operated by a knowledge worker that is interested in the data, the network architecture comprising: an information tier to collect data from the plurality of IMDs; a first analyzing tier to analyze the data collected from the information source layer utilizing a first set of analytical capabilities; and a second analyzing tier, separate from the first analyzing tier, to further analyze the data analyzed by the first analyzing tier, wherein the second analyzing tier utilizes a second set of analytical capabilities that are not included in the first set of analytical capabilities.

Preferably, the second analyzing tier is configured to communicate a result of the second analyzing to one or more of the plurality of IMDs. Preferably, the information tier includes the plurality of IMDs; the first analyzing tier includes one or more of the computing devices configured as intermediate computing devices; and the second analyzing tier includes one of the computing devices configured as a endpoint device.

The second analyzing layer may be configured to request additional data from the information source layer that was not included in the data provided by the first analyzing layer. Preferably, at least one of the first and second analyzing tier form a communication for notifying a designated user of a result of the respective analysis. The information layer may further comprise an external medical device.

A hierarchical data storage and analysis system for implantable medical devices is described. The system may be implemented through a plurality of intelligent agents that are executed at each tier of the hierarchy to perform defined tasks. For example, execution of the agents may be utilized to manage data based on the capabilities of each tier of the hierarchy, such as analytical capabilities to analyze patient data, processing capabilities, memory capabilities, communication capabilities, and so forth.

The invention may be carried into practice in various ways and some embodiments will now be described by way of example, with reference to the accompanying drawings, in which:

Fig. 1 is a diagrammatic illustration of a cardiac therapy network architecture with an implantable medical device (IMD), configured as an implantable cardiac therapy device, connected to a network of computing devices;

Fig. 2 shows a hierarchy in an exemplary implementation in which the hierarchy is defined by analytical capabilities of each of the computing devices included in the hierarchy;

Fig. 3 is a simplified illustration of the IMD of Fig. 1 in electrical communication with a patient's heart for monitoring heart activity and/or delivering stimulation therapy;

Fig. 4 is a functional block diagram of the exemplary IMD of Fig. 3;

Fig. 5 is a functional block diagram of an exemplary computing device that may be used in the network systems of the network architecture;

Fig. 6 is a block diagram depicting an agent hierarchy having four separate tiers, each of the tiers illustrated as having an agent to manage data in the respective tier;

Fig. 7 is a block diagram showing an agent hierarchy having exemplary agents for inclusion on one or more of the computing devices of the hierarchy;

Fig. 8 is a flow chart depicting a process for managing data in an agent hierarchy based on analytical capabilities of one or more computing devices included in the network system;

Fig. 9 is a flow chart depicting a process for managing data in an agent hierarchy based on processing capabilities of one or more computing devices included in the network system;

Fig. 10 is a flow chart depicting a process for managing data in an agent hierarchy based on data criticality and memory capabilities of one or more computing devices included in the network system;

Fig. 11 shows an agent hierarchy implemented in a network system in which data is managed through execution of a plurality of agents by respective computing devices;

Fig. 12 is a flow chart depicting a process for managing critical data in a network system;

Fig. 13 shows an agent hierarchy implemented in a network system in which data is communicated from the endpoint device through tiers of the hierarchy to the IMD through execution of a plurality of agents by respective computing devices; and

Fig. 14 shows an agent hierarchy implemented in a network system in which data is communicated from a plurality of IMDs and external medical devices through tiers of the hierarchy to the endpoint device through execution of a plurality of agents by respective computing devices.

The following description sets forth an implantable medical device (IMD) network architecture in which data collected from an IMD is managed for communication, analysis, and/or storage by computing devices included in the network architecture. For example, the network architecture may include a variety of computing devices such as the IMD, one or more intermediate computing devices, and an endpoint device. One or more of the computing devices may have different analytical capabilities to analyze the data, may have differing amount of data storage, may have different respective amounts of processing resources, may utilize different respective patient diagnostic algorithms to analyze the data, and so forth. Accordingly, the network architecture may be configured as one or more hierarchies that reflect the different capabilities of each of the computing devices such that analysis and storage of the data is optimized.

### Cardiac Therapy Network

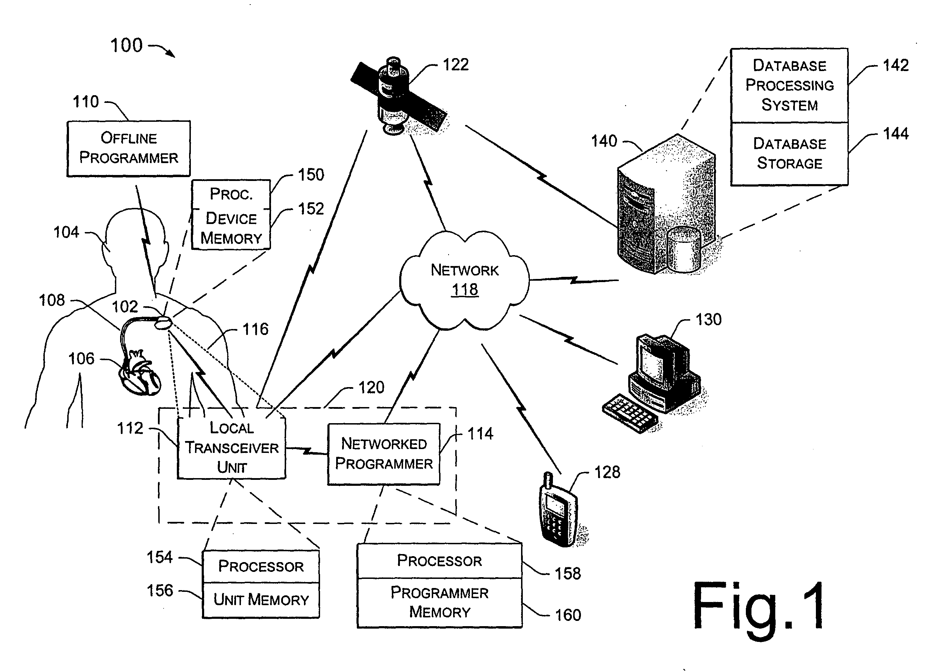

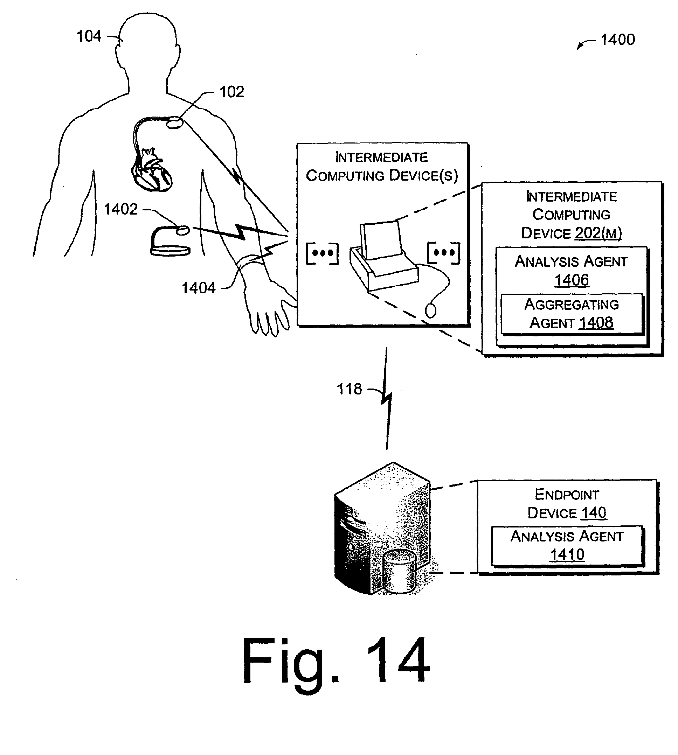

Fig. 1 shows an exemplary network architecture 100 that includes an implantable medical device (IMD) 102, which is represented pictorially as an implantable cardiac therapy device (ICTD), coupled to a network of computing devices. The IMD 102 is illustrated as being implanted in a human patient 104. The IMD 102 is also illustrated in Fig. 1 as being in electrical communication with a patient's heart 106 by way of multiple leads 108 suitable for monitoring cardiac activity and/or delivering multi-chamber stimulation and shock therapy. Although the IMD 102 is illustrated as an ICTD, the IMD 102 may be configured in a variety of ways, such as implantable cardiac monitors that monitor heart activity, implantable neurological devices that monitor and stimulate nerves, and other implantable devices used for diagnostic and/or therapeutic purposes, further discussion of which may be found in relation to Fig. 14.

The IMD 102 may communicate with a standalone or offline programmer 110 via short-range telemetry technology. The offline programmer 110 is equipped with a wand that, when positioned proximal to the IMD 102, communicates with the IMD 102 through a magnetic coupling.

The IMD 102 can alternatively, or additionally, communicate with a local transceiver. The local transceiver 112 may be a device that resides on or near the patient, such as an electronic communications device that is worn by the patient or is situated on a structure within the room or residence of the patient. The local transceiver 112 communicates with the IMD 102 using short-range telemetry or longer-range high-frequency-based telemetry, such as RF (radio frequency) transmissions. Alternatively, the local transceiver 112 may be incorporated into the IMD 102, as represented by dashed line 116. In this case, the IMD includes a separate and isolated package area that accommodates high-frequency transmissions without disrupting operation of the monitoring and stimulation circuitry.

Depending upon the implementation and transmission range, the local transceiver 112 can be in communication with various other devices of the network architecture 100. One possible implementation is for the local transceiver 112 to transmit information received from the IMD 102 to a networked programmer 114, which is connected to network 118. The networked programmer 114 is similar in operation to standalone programmer 110, but differs in that it is connected to the network 118. The networked programmer 114 may be local to, or remote from, the local transceiver 112; or alternatively, the local transceiver 112 may be incorporated into the networked programmer 114, as represented by dashed line 120.

Another possible implementation is for the local transceiver to be connected directly to the network 118 for communication with remote computing devices and/or programmers. Still another possibility is for the local transceiver 112 to communicate with the network 118 via wireless communication, such as via a satellite system 122.

The network 118 may be implemented by one or more different types of networks (e.g., Internet, local area network, wide area network, telephone, cable, satellite, etc.), including wire-based technologies (e.g., telephone line, cable, fiber optics, etc.) and/or wireless technologies (e.g., RF, cellular, microwave, IR, wireless personal area network, etc.). The network 118 can be configured to support any number of different protocols, including HTTP (HyperText Transport Protocol), TCP/IP (Transmission Control Protocol/Internet Protocol), WAP (Wireless Application Protocol), Bluetooth, and so on.

The network architecture 100 facilitates distribution of data among the IMD 102, the local transceiver unit 112, the networked programmer 114, and one or more hierarchical endpoint devices, represented pictorially in Fig. 1 as a patient notification device 128, a healthcare worker notification device 130, and a database server 140. The endpoint devices can be implemented using a wide variety of other computing devices, ranging from small handheld computers or portable digital assistants (PDAs) carried by physicians to workstations or mainframe computers with large storage capabilities.

Data gathered from the IMD 102 may be integrated, processed, and distributed by each of the computing devices (e.g., the IMD 102, offline programmer 110, networked programmer 114, patient notification device 128, knowledge worker notification device 130, and database server 140) for viewing by knowledge workers. The endpoint devices may maintain and store the data from the IMD 102, and prepare the data for efficient presentation to knowledge workers. Additionally, the endpoint devices may be utilized in a variety of ways. For example, the endpoint devices may be utilized by healthcare providers to store and process patient records. A manufacturer may utilize the endpoint device configured as a computer system that tracks data returned from the IMD 102. Additionally, clinical groups may utilize the endpoint device to store and analyze data across patient populations. Further, regulatory agencies may utilize the endpoint device to register and track healthcare risk data for a plurality of IMDs.

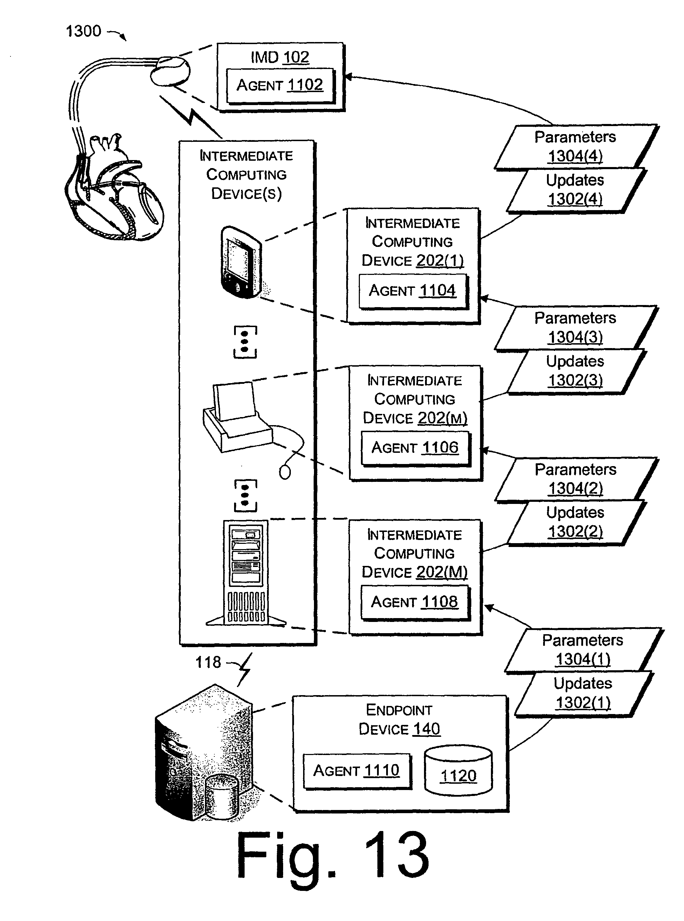

The network architecture 100 supports two-way communication. Not only is data collected from the IMD 102 and distributed to the various computing devices, but also data may be returned from these computing devices to the networked programmer 114 and/or the local transceiver 112 for communication back to the IMD 102. Information returned to the IMD 102 may be used to adjust operation of the device, update software executed on the device, and/or modify therapies being applied by the device. Such information may be imparted to the IMD 102 automatically, without the patient's knowledge. Further discussion of adjustment of parameters used in patient diagnostic algorithms and software upgrades may be found in relation to Fig. 13.

Additionally, data may be sent to a patient notification device 128 to notify the patient of some event or item. The patient notification device 128 can be implemented in a number of ways including, for example, as a telephone, a cellular phone as illustrated, a pager, a PDA (personal digital assistant), a dedicated patient communication device, a computer, an alarm, and so on. Notifications may be as simple as an instruction to sound an alarm to inform the patient to call into the healthcare providers, or as complex as HTML-based pages with graphics and textual data to educate the patient. Notification messages sent to the patient notification device 128 can contain essentially any type of information related to therapeutic purposes and/or device operation. Such information might include new studies released by clinical groups pertaining to device operation and patient activity (e.g., habits, diets, exercise, etc.), recall notices or operational data from the manufacturer, patient-specific instructions sent by the healthcare providers, or warnings published by regulatory groups.

Notifications can also be sent from the knowledge worker to the patient. Notification device 130 might include, for example, one or more computing devices designed to create and deliver notification messages on behalf of the knowledge workers. The notification system 130 delivers the messages in formats supported by the various types of patient notification devices 128. For instance, if the patient carries a pager, a notification message might consist of a simple text statement in a pager protocol. For a more sophisticated wireless-enabled PDA or Internet-oriented cellular phone, messages might contain more than text data and be formatted using WAP formats.

Each of the computing devices illustrated in Fig. 1 possess differing capabilities, such as different analytical capabilities, different processing functionality and/or different storage capacities. For example, IMD 102 may be configured with a processor 150 and memory 152 of limited capabilities, while database server 140 has a database processing system 142 and database storage 144 of extensive capabilities. Intermediate devices may have different sets of capabilities as compared to the IMD 102 and database server 140. Here, local transceiver unit 112 has a processor 154 and a memory 156, and networked programmer 114 has a processor 158 and a memory 160. The processors 154 and 158 and memories 156 and 160 can range from more enhanced capabilities in comparison with the server to capabilities that fall somewhere between the IMD and server, to less capabilities than the IMD.

Taken together as a single architecture, the computing devices form a hierarchy of varying analytical capabilities for processing and storing data collected by the IMD 102. Data is migrated among the devices to take advantage of these capability differences depending upon defmed sets of heuristics.

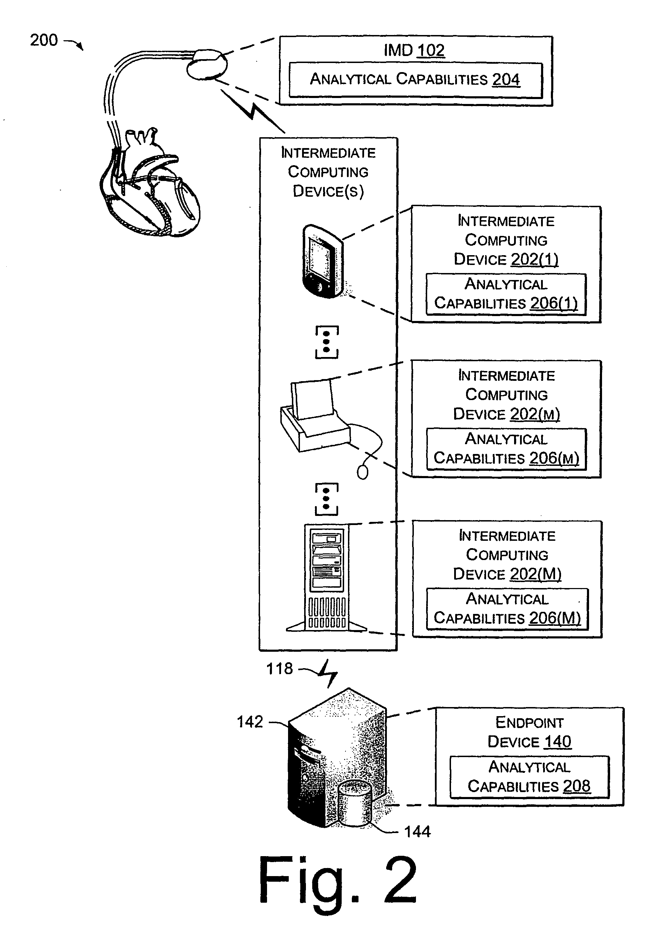

Fig. 2 shows a hierarchy 200 in an exemplary implementation in which the hierarchy 200 is defined by analytical capabilities of each of the computing devices included in the hierarchy 200. The hierarchy 200 includes a computing device configured as the IMD 102 of Fig. 1 and another computing device configured as an endpoint device 140. The hierarchy 200 also includes a plurality of intermediate computing devices 202(1), ..., 202(m), ..., 202(M). One or more of the plurality of intermediate computing devices 202(1)-202(M) are communicatively coupled to the IMD 102. Additionally, one or more of the plurality of intermediate computing devices 202(1)-202(M) are communicatively coupled to the endpoint device 140 over the network 118.

Each of the computing devices in the hierarchy 200 has respective analytical capabilities. The IMD 102 includes analytical capabilities 204, each of the intermediate computing devices 202(1)-202(M) includes respective analytical capabilities 206(1)-206(M), and the endpoint device 140 also includes analytical capabilities 208. However, the analytical capabilities of each of the computing devices may differ.

The analytical capabilities 204 of the IMD 102, for example, may be limited by hardware and software capabilities of the IMD 102. For instance, the IMD 102 may have a limited amount of memory. Often, this memory is shared by diagnostic data and software that is executed on the IMD 102 to control the functionality of the IMD 102. Thus, there is a continuing need for more memory for use by software of the IMD 102 to provide ever increasing levels of functionality and for use in storing additional diagnostic information to help a physician in programming the IMD 102 to settings that are optimal for a given patient. Adding more memory to the IMD 102, however, increases both the power consumption and the size of the device.

Additionally, patient data stored in the memory is telemetered out of the IMD 102 to a programmer for examination by a knowledge worker (e.g. a physician, nurse, physician's assistant, etc.). Telemetry speeds, however, may be limited by the amount of current that can be drawn from the battery of the IMD 102. Telemetry speeds may also be limited due to attenuation of high frequency signals when utilized to communicate the data through the patient's body and/or a casing of the IMD 102, which may pose limitations in which frequencies can be used for communication. The processing capabilities within the IMD 102 may also be limited due to the power and space considerations in the design of the IMD 102.

Therefore, to provide additional analytical capabilities to analyze data collected by the IMD 102, the hierarchy 200 employs successive tiers, with one or more successive tiers in the hierarchy 200 from the IMD 102 having additional analytical capabilities. For example, the IMD 102 may communicate the data to an intermediate computing device 202(1) which is represented pictorially as an external patient device for being worn by the patient. The intermediate computing device 202(1) includes respective analytical capabilities 206(1) that are not included in the analytical capabilities 204 of the IMD 102. For instance, intermediate computing device 202(1) may include additional hardware resources (e.g., processor, memory, battery power, etc.), and/or software resources (e.g., patient diagnostic algorithms, device diagnostic algorithms, etc.) because the intermediate computing device 202(1) does not have the same space and power limitations encountered by the IMD 102 when implanted in the patient's body. The intermediate computing device 202(1), however, may be configured to be worn by the patient, and therefore have a somewhat limited size.

Intermediate computing device 202(m) is represented pictorially as a programmer, which may or may not correspond to the offline programmer 110 and/or the networked programmer 114 of Fig. 1. The intermediate computing device 202(m) is designed for mobile applications such that it may be transported for retrieving data from the IMD 102. As such, the intermediate computing device 202(m) is not as limited by size considerations as the intermediate computing device 202(1) and the IMD 102. Thus, analytical capabilities 206(m) of the intermediate computing device 202(1) may include additional capabilities that are not available to the intermediate computing device 202(1) and the IMD 102. For instance, the intermediate computing device 202(m) may have additional memory resources such that it may store a greater amount of patient data and employ additional diagnostic algorithms to analyze the data from the IMD 102.

Likewise, intermediate computing device 202(M) is represented pictorially as a server that is not configured for mobile applications. Therefore, intermediate computing device 202(M) may include analytical capabilities 206(M) that are not available to any of the previously described computing devices in the hierarchy 200, i.e. the IMD 102 and the intermediate computing devices 202(1), 202(m).

The endpoint device 140 in this implementation includes the data processing system 124 and database storage 126 of Fig. 1. Thus, the endpoint device 140 may provide a central repository for data relating to one or more patients. The endpoint device 140, for instance, may store data that was previously output by the IMD 102 as well as current data output by the IMD 102. The increased amount of patient data may increase the analytical capabilities 208 of the endpoint device 140. For example, the endpoint device 140 may execute patient diagnostic algorithms that utilize heuristics to track patient progress. Thus, a greater overall "picture" of the patient's condition may be provided through examination of the increased amounts of patient data. The endpoint device 140 may also include increased hardware and/or software capabilities over other computing devices in the hierarchy 200, such as increased processing resources, increased network connection bandwidth, and so on.

Thus, in the implementation shown in Fig. 2, the hierarchy 200 is defined by a plurality of "tiers" having additional analytical capabilities that may be provided through a combination of hardware, software, and network resources of each of the computing devices in the hierarchy 200. Although in this implementation five tiers are shown with each tier corresponding to a different computing device, the hierarchy 200 can be built to include two to "n" tiers based on the desired capabilities of a network system implementing the hierarchy. Additionally, although single computing devices are shown for each tier in the hierarchy 200, the tiers may correspond to logical tiers based on the capabilities of collections of devices. For example, the endpoint device 140 may employ a plurality of servers, e.g. a server farm, that provides the analytical capabilities 208.

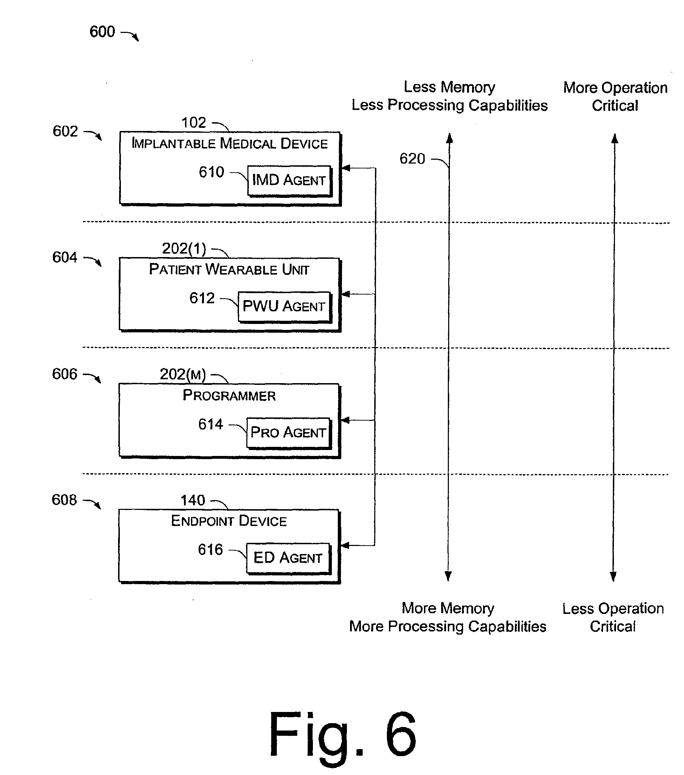

To manage data in the hierarchy 200, both from the IMD 102 through the intermediate computing devices 202(1)-202(M) to the endpoint device 140, and vice versa, each of the computing devices may execute one or more agents. The agents, when executed, may manage distribution of the data based on capabilities of the respective computing device, further description of which may be found starting in relation to Fig. 6.

### Exemplary IMD

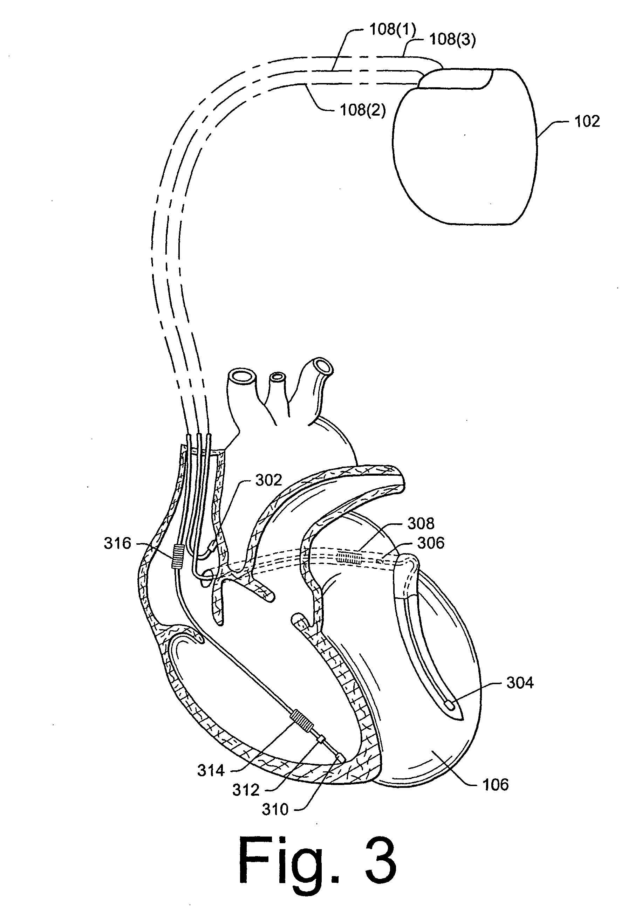

Fig. 3 shows an exemplary IMD 102 in electrical communication with a patient's heart 106 for monitoring heart activity and/or delivering stimulation therapy, such as pacing or defibrillation therapies. The IMD 102 is in electrical communication with a patient's heart 106 by way of three leads 302(1)-(3). To sense atrial cardiac signals and to provide right atrial chamber stimulation therapy, the IMD 102 is coupled to an implantable right atrial lead 108(1) having at least an atrial tip electrode 302, which typically is implanted in the patient's right atrial appendage. To sense left atrial and ventricular cardiac signals and to provide left chamber pacing therapy, the IMD 102 is coupled to a coronary sinus lead 108(2) designed for placement in the coronary sinus region via the coronary sinus. The coronary sinus lead 108(2) positions a distal electrode adjacent to the left ventricle and/or additional electrode(s) adjacent to the left atrium. An exemplary coronary sinus lead 108(2) is designed to receive atrial and ventricular cardiac signals and to deliver left ventricular pacing therapy using at least a left ventricular tip electrode 304, left atrial pacing therapy using at least a left atrial ring electrode 306, and shocking therapy using at least a left atrial coil electrode 308.

The IMD 102 is also shown in electrical communication with the patient's heart 106 by way of an implantable right ventricular lead 108(3) having, in this implementation, a right ventricular tip electrode 310, a right ventricular ring electrode 312, a right ventricular (RV) coil electrode 314, and an SVC coil electrode 316. Typically, the right ventricular lead 108(3) is transvenously inserted into the heart 106 to place the right ventricular tip electrode 310 in the right ventricular apex so that the RV coil electrode 314 will be positioned in the right ventricle and the SVC coil electrode 316 will be positioned in the superior vena cava. Accordingly, the right ventricular lead 108(3) is capable of receiving cardiac signals, and delivering stimulation in the form of pacing and shock therapy to the right ventricle.

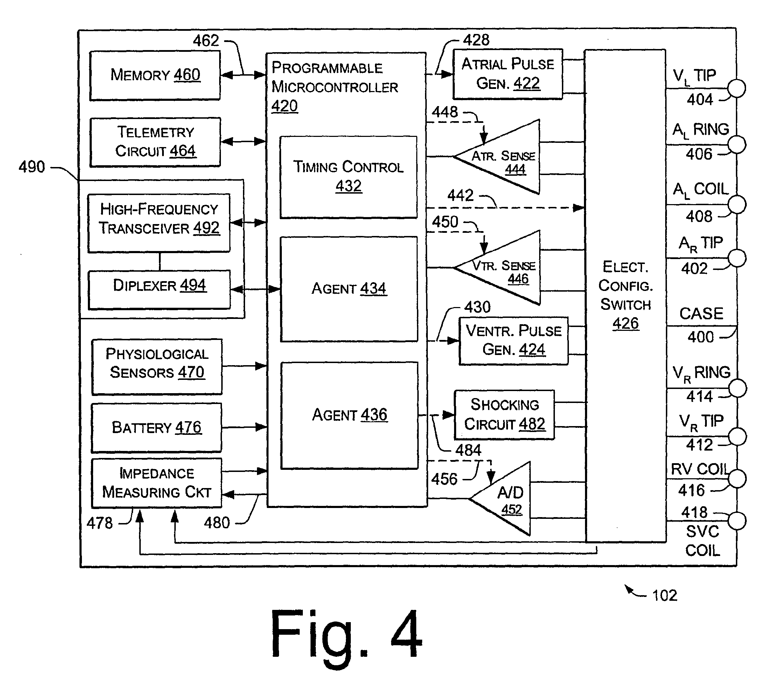

Fig. 4 shows an exemplary, simplified block diagram depicting various components of the IMD 102. The IMD 102 can be configured to perform one or more of a variety of functions including, for example, monitoring heart activity, monitoring patient activity, and treating fast and slow arrhythmias with stimulation therapy that includes cardioversion, defibrillation, and pacing stimulation. While a particular multi-chamber device is shown, it is to be appreciated and understood that this is done for illustration purposes.

The circuitry is housed in housing 400, which is often referred to as the "can", "case", "encasing", or "case electrode", and may be programmably selected to act as the return electrode for unipolar modes. Housing 400 may further be used as a return electrode alone or in combination with one or more of the coil electrodes for shocking purposes. Housing 400 further includes a connector (not shown) having a plurality of terminals 402, 404, 406, 408, 412, 414, 416, and 418 (shown schematically and, for convenience, the names of the electrodes to which they are connected are shown next to the terminals).

To achieve right atrial sensing and pacing, the connector includes at least a right atrial tip terminal (A_{R} TIP) 402 adapted for connection to the atrial tip electrode 302. To achieve left chamber sensing, pacing, and shocking, the connector includes at least a left ventricular tip terminal (V_{L} TIP) 404, a left atrial ring terminal (A_{L} RING) 406, and a left atrial shocking terminal (A_{L} COIL) 408, which are adapted for connection to the left ventricular ring electrode 304, the left atrial ring electrode 306, and the left atrial coil electrode 308, respectively. To support right chamber sensing, pacing, and shocking, the connector includes a right ventricular tip terminal (V_{R} TIP) 412, a right ventricular ring terminal (V_{R} RING) 414, a right ventricular shocking terminal (RV COIL) 416, and an SVC shocking terminal (SVC COIL) 418, which are adapted for connection to the right ventricular tip electrode 310, right ventricular ring electrode 312, the RV coil electrode 314, and the SVC coil electrode 316, respectively.

At the core of the IMD 102 is a programmable microcontroller 420 that controls various operations of the ICTD, including cardiac monitoring and stimulation therapy. Microcontroller 420 includes a microprocessor (or equivalent control circuitry), RAM and/or ROM memory, logic and timing circuitry, state machine circuitry, and I/O circuitry. Microcontroller 420 includes the ability to process or monitor input signals (data) as controlled by a program code stored in a designated block of memory. Any suitable microcontroller 420 may be used.

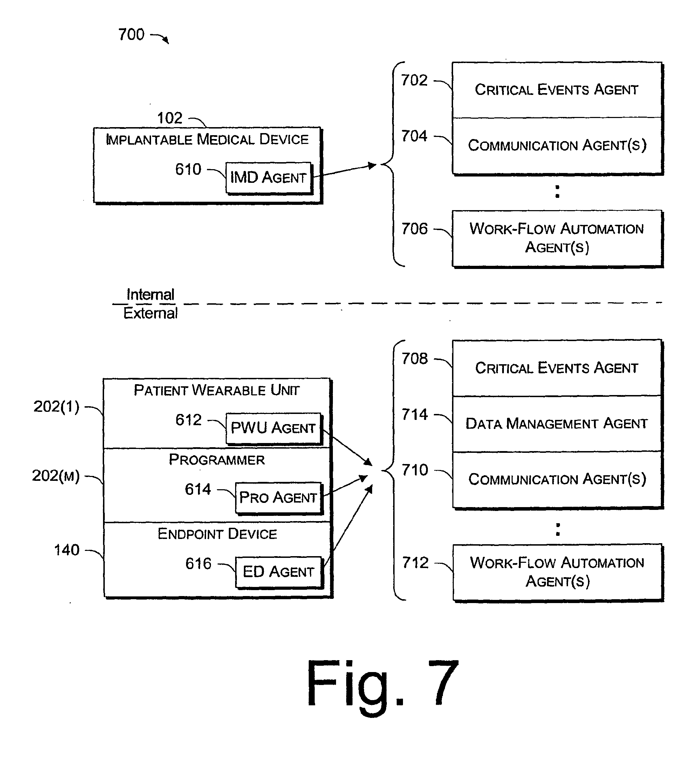

For discussion purposes, microcontroller 420 is illustrated as including timing control circuitry 432 to control the timing of the stimulation pulses (e.g., pacing rate, atrio-ventricular (AV) delay, atrial interconduction (A-A) delay, or ventricular interconduction (V-V) delay, etc.) as well as to keep track of the timing of refractory periods, blanking intervals, noise detection windows, evoked response windows, alert intervals, marker channel timing, and so on. Microcontroller 420 may further include a plurality of agents 434, 436. The agents, when executed, can be utilized to control functionality of the device 102. The agents 434, 436 may be implemented in hardware as part of the microcontroller 420, or as software/firmware instructions programmed into the device and executed on the microcontroller 420 during certain modes of operation. Further discussion of agent functionality may be found in relation to Figs. 6 and 7.

The IMD 102 further includes an atrial pulse generator 422 and a ventricular pulse generator 424 that generate pacing stimulation pulses for delivery by the right atrial lead 108(1), the coronary sinus lead 108(2), and/or the right ventricular lead 108(3) via an electrode configuration switch 426. It is understood that in order to provide stimulation therapy in each of the four chambers of the heart, the atrial and ventricular pulse generators, 422 and 424, may include dedicated, independent pulse generators, multiplexed pulse generators, or shared pulse generators. The pulse generators 422 and 424 are controlled by the microcontroller 420 via appropriate control signals 428 and 430, respectively, to trigger or inhibit the stimulation pulses.

The electronic configuration switch 426 includes a plurality of switches for connecting the desired electrodes to the appropriate I/O circuits, thereby providing complete electrode programmability. Accordingly, switch 426, in response to a control signal 442 from the microcontroller 420, determines the polarity of the stimulation pulses (e.g., unipolar, bipolar, combipolar, etc.) by selectively closing the appropriate combination of switches (not shown).

Atrial sensing circuits 444 and ventricular sensing circuits 446 may also be selectively coupled to the right atrial lead 108(1), coronary sinus lead 108(2), and the right ventricular lead 108(3), through the switch 426 to detect the presence of cardiac activity in each of the four chambers of the heart. Accordingly, the atrial (ATR. SENSE) and ventricular (VTR. SENSE) sensing circuits, 444 and 446, may include dedicated sense amplifiers, multiplexed amplifiers, or shared amplifiers. Each sensing circuit 444 and 446 may further employ one or more low power, precision amplifiers with programmable gain and/or automatic gain control, bandpass filtering, and a threshold detection circuit to selectively sense the cardiac signal of interest. The automatic gain control enables the IMD 102 to deal effectively with the difficult problem of sensing the low amplitude signal characteristics of atrial or ventricular fibrillation. Switch 426 determines the "sensing polarity" of the cardiac signal by selectively closing the appropriate switches. In this way, the clinician may program the sensing polarity independent of the stimulation polarity.

The outputs of the atrial and ventricular sensing circuits 444 and 446 are connected to the microcontroller 420 which, in turn, is able to trigger or inhibit the atrial and ventricular pulse generators 422 and 424, respectively, in a demand fashion in response to the absence or presence of cardiac activity in the appropriate chambers of the heart. The sensing circuits 444 and 446 receive control signals over signal lines 448 and 450 from the microcontroller 420 for purposes of controlling the gain, threshold, polarization charge removal circuitry (not shown), and the timing of any blocking circuitry (not shown) coupled to the inputs of the sensing circuits 444 and 446.

Cardiac signals are also applied to inputs of an analog-to-digital (A/D) data acquisition system 452. The data acquisition system 452 is configured to acquire intracardiac electrogram signals, convert the raw analog data into a digital signal, and store the digital signals for later processing and/or telemetric transmission to an external device 454. The data acquisition system 452 is coupled to the right atrial lead 108(1), the coronary sinus lead 108(2), and the right ventricular lead 108(3) through the switch 426 to sample cardiac signals across any pair of desired electrodes.

The data acquisition system 452 may be coupled to the microcontroller 420, or other detection circuitry, to detect an evoked response from the heart 106 in response to an applied stimulus, thereby aiding in the detection of "capture". Capture occurs when an electrical stimulus applied to the heart is of sufficient energy to depolarize the cardiac tissue, thereby causing the heart muscle to contract. The microcontroller 420 detects a depolarization signal during a window following a stimulation pulse, the presence of which indicates that capture has occurred. The microcontroller 420 enables capture detection by triggering the ventricular pulse generator 424 to generate a stimulation pulse, starting a capture detection window using the timing control circuitry 432 within the microcontroller 420, and enabling the data acquisition system 452 via control signal 456 to sample the cardiac signal that falls in the capture detection window and, based on the amplitude, determines if capture has occurred.

The microcontroller 420 is further coupled to a memory 460 by a suitable data/address bus 462, wherein the programmable operating parameters used by the microcontroller 420 are stored and modified, as required, in order to customize the operation of the implantable device 102 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 106 within each respective tier of therapy. The operating parameters may be updated through communication with an external device, further discussion of which may be found in relation to Fig. 13. With memory 460, the IMD 102 is able to sense and store a relatively large amount of data (e.g., from the data acquisition system 452), which may transmitted to the external network of knowledge workers for subsequent analysis.

Operating parameters of the IMD 102 may be non-invasively programmed into the memory 460 through a telemetry circuit 464 in telemetric communication with an external device, such as a programmer 110 or local transceiver 112. The telemetry circuit 464 advantageously allows intracardiac electrograms and status information relating to the operation of the device 102 (as contained in the microcontroller 420 or memory 460) to be sent to the external devices.

The IMD 102 can further include one or more physiologic sensors 470, commonly referred to as "rate-responsive" sensors because they are typically used to adjust pacing stimulation rate according to the exercise state of the patient. However, the physiological sensor 470 may further be used to detect changes in cardiac output, changes in the physiological condition of the heart, or diurnal changes in activity (e.g., detecting sleep and wake states, detecting position or postural changes, etc.). Accordingly, the microcontroller 420 responds by adjusting the various pacing parameters (such as rate, AV Delay, V-V Delay, etc.) at which the atrial and ventricular pulse generators, 422 and 424, generate stimulation pulses. While shown as being included within the device 102, it is to be understood that the physiologic sensor 470 may also be external to the device 102, yet still be implanted within or carried by the patient. Examples of physiologic sensors that may be implemented in device 102 include known sensors that, for example, sense respiration rate and/or minute ventilation, pH of blood, ventricular gradient, and so forth.

The IMD 102 additionally includes a battery 476 that provides operating power to all of circuits shown in Fig. 4. If the device 102 is configured to deliver pacing or shocking therapy, the battery 476 is capable of operating at low current drains for long periods of time (e.g., preferably less than 10 µA), and is capable of providing high-current pulses (for capacitor charging) when the patient requires a shock pulse (e.g., preferably, in excess of 2 A, at voltages above 2 V, for periods of 10 seconds or more). The battery 476 also desirably has a predictable discharge characteristic so that elective replacement time can be detected. As one example, the device 102 employs lithium/silver vanadium oxide batteries.

The IMD 102 can further include magnet detection circuitry (not shown), coupled to the microcontroller 420, to detect when a magnet is placed over the device 102. A magnet may be used by a clinician to perform various test functions of the device 102 and/or to signal the microcontroller 420 that the external programmer is in place to receive or transmit data to the microcontroller 420 through the telemetry circuits 464.

The IMD 102 further includes an impedance measuring circuit 478 that is enabled by the microcontroller 420 via a control signal 480. Uses for an impedance measuring circuit 478 include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 478 is advantageously coupled to the switch 426 so that any desired electrode may be used.

In the case where the IMD 102 is intended to operate as an implantable cardioverter/defibrillator (ICD) device, it detects the occurrence of an arrhythmia, and automatically applies an appropriate electrical shock therapy to the heart aimed at terminating the detected arrhythmia. To this end, the microcontroller 420 further controls a voltage delivery circuit or shock circuit 482 by way of a control signal 484. The shocking circuit 482 generates shocking pulses of low (up to 0.5 Joules), moderate (0.5 - 10 Joules), or high energy (11 to 40 Joules), as controlled by the microcontroller 420. Such shocking pulses are applied to the patient's heart 106 through at least two shocking electrodes, and as shown in this implementation, selected from the left atrial coil electrode 308, the RV coil electrode 314, and/or the SVC coil electrode 316. As noted above, the housing 400 may act as an active electrode in combination with the RV coil electrode 314, or as part of a split electrical vector using the SVC coil electrode 316 or the left atrial coil electrode 308 (i.e., using the RV electrode as a common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 Joules), delivered asynchronously (since R-waves may be too disorganized), and pertaining exclusively to the treatment of fibrillation. Accordingly, the microcontroller 420 is capable of controlling the synchronous or asynchronous delivery of the shocking pulses.

The IMD 102 is further designed with the ability to support high-frequency wireless communication, typically in the radio frequency (RF) range. The IMD 102 is equipped with a high-frequency transceiver 492 and a diplexer 494. High-frequency signals received by a dedicated antenna 496, or via leads 108, are passed to the transceiver 492 directly, or via diplexer 494. The high-frequency transceiver 492 may be configured to operate on one or a few frequencies. Alternatively, the transceiver 492 may include a tuner 496 that tunes to various frequencies when attempting to establish communication links with the external communication device (e.g., programmer, local transceiver, etc.).

In one implementation, the high-frequency circuitry may be contained within a secondary, isolated casing 490 to enable handling of high-frequency signals in isolation from the cardiac therapy circuitry. In this manner, the high-frequency signals can be safely received and transmitted, thereby improving telemetry communication, without adversely disrupting operation of the other device circuitry.

### Exemplary Computing Device

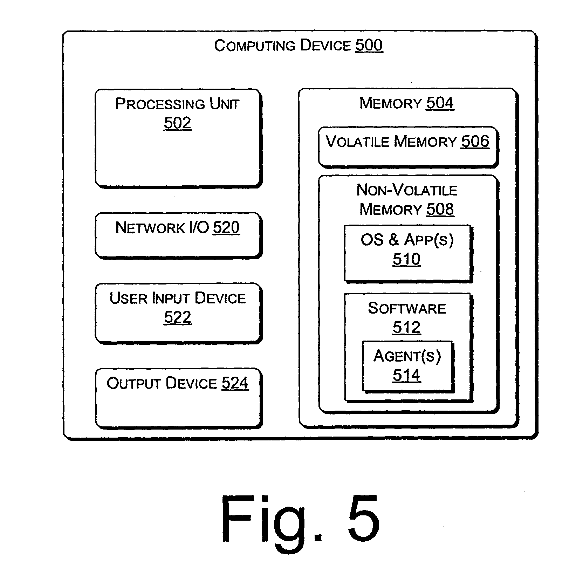

Fig. 5 shows an exemplary computing device 500 employed in the network architecture 100. It includes a processing unit 502 and memory 504. Memory 504 includes both volatile memory 506 (e.g., RAM) and non-volatile memory 508 (e.g., ROM, EEPROM, Flash, disk, optical discs, persistent storage, etc.). An operating system and various application programs 510 and software 512 are stored in non-volatile memory 508. When a program is running, various instructions are loaded into volatile memory 506 and executed by processing unit 502. Examples of software 512 may include one or more agents 514 that may be stored and executed on the computing device 500, further discussion of which may be found in relation to Figs. 6 and 7.

The computing device 500 may further be equipped with a network I/O connection 520 to facilitate communication with a network. The network I/O 520 may be a wire-based connection (e.g., network card, modem, etc.) or a wireless connection (e.g., RF transceiver, Bluetooth device, etc.). The computing device 500 may also include a user input device 522 (e.g., keyboard, mouse, stylus, touch pad, touch screen, voice recognition system, etc.) and an output device 524 (e.g., monitor, LCD, speaker, printer, etc.).

Various aspects of the methods and systems described throughout this disclosure may be implemented in computer software or firmware as computer-executable instructions. When executed, these instructions direct the computing device (alone, or in concert with other computing devices of the system) to perform various functions and tasks that enable the network architecture 100.

### Agent Hierarchy

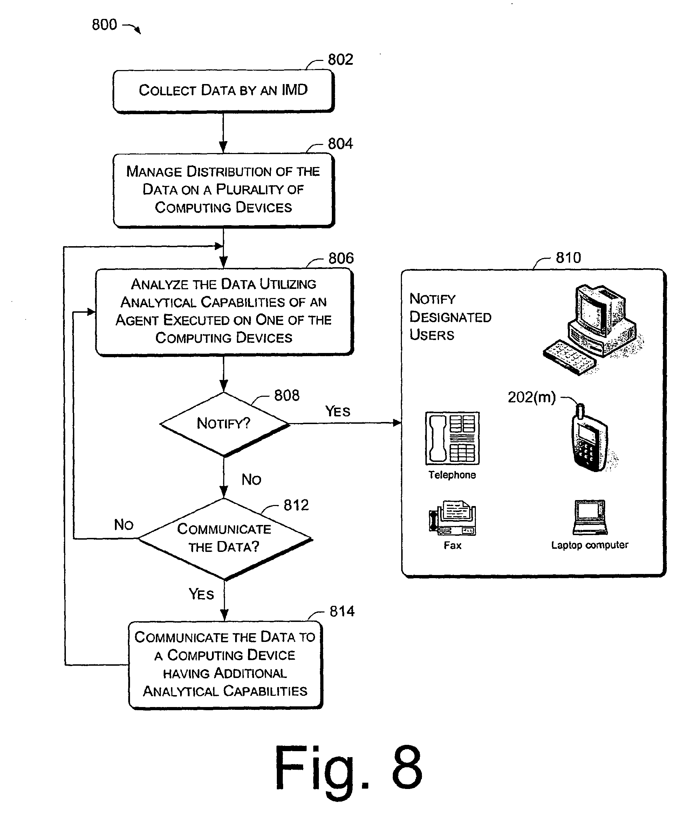

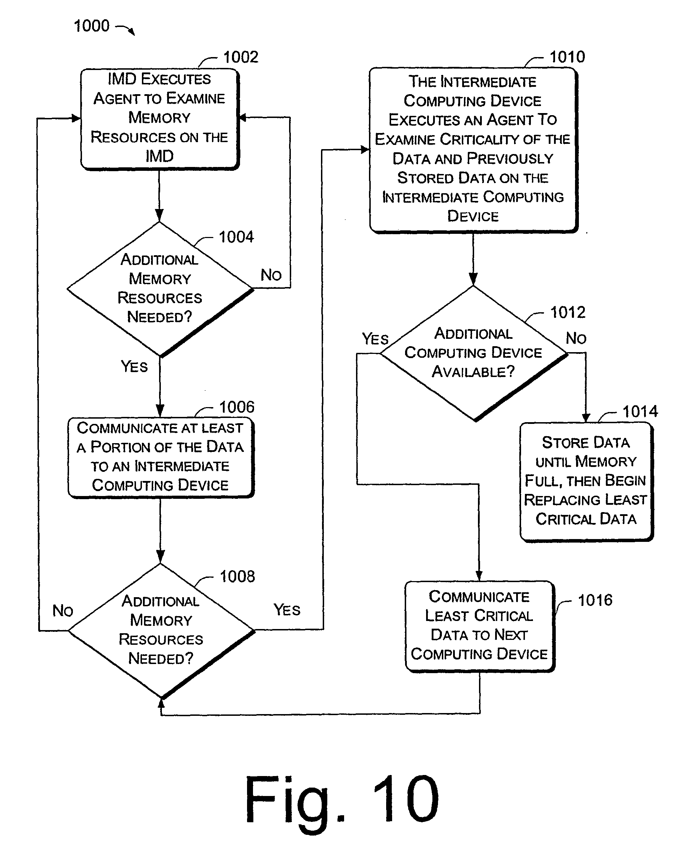

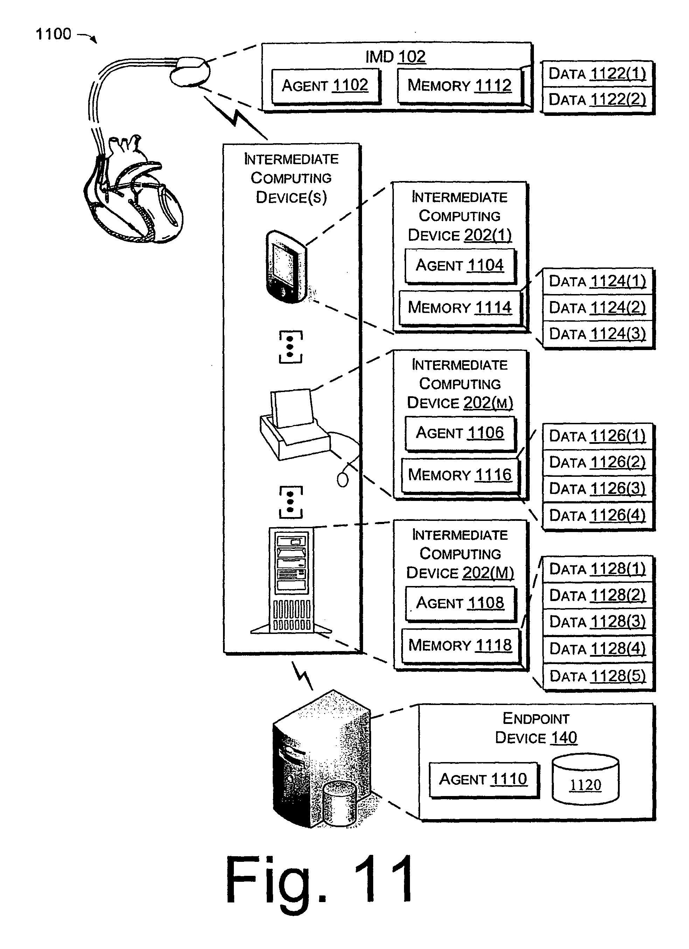

The agent hierarchy provides a mechanism for managing collection, storage, and/or analysis of data from one or more IMDs. The agent hierarchy may employ a variety of hierarchies that are configured to address capabilities of computing devices of a network system implementing the hierarchy. Examples of capabilities may include analytical capabilities, processing capabilities, memory capabilities, network capabilities, and so on, examples of which are described in greater detail in relation to Figs. 8, 9 and 10, respectively. Additionally, the agent hierarchy may dynamically address capabilities of the computing devices in the network during "runtime", further discussion of which may be found in relation to Fig. 11. The network architecture may also manage data based on "criticality" of the data, further discussion of which may be found in relation to Figs. 6 and 10.

Fig. 6 is a block diagram depicting an agent hierarchy 600 having four separate tiers 602-608, each of the tiers illustrated as having an agent to manage data in the respective tier. A first tier 602 includes the IMD 102 and is utilized to collect data describing a patient, such as diagnostic data describing the patient and so on. Second and third tiers 604, 606 include the intermediate computing devices 202(1), 202(m) of Fig. 2 that are represented pictorially as a patient wearable unit and a programmer, respectively. A fourth tier 608 includes the endpoint device 140.

As previously described, each of the tiers 602-608 may have different capabilities. In Fig. 6, a solid arrow 620 illustrates the varying processing and memory capabilities across each of the tiers 602-608. For example, the second tier 604 has more processing and memory capabilities than the first tier 602, the third tier 606 has more processing and memory capabilities than the second tier 604, and so on.

Each tier 602-608 includes an agent 610-616 that addresses capabilities of the respective tier 602-608. For example, the IMD 102 includes an IMD agent 610 that, when executed, manages data on the IMD 102 according to the memory and processing capabilities of the IMD 102. The intermediate computing device 202(1), represented as a patient wearable unit, includes a patient wearable unit agent 612 that, when executed, manages data on the intermediate computing device 202(1) according to the memory and processing capabilities of the intermediate computing device 202(1). Likewise, the intermediate computing device 202(M) includes a programmer agent 614 and the endpoint device 140 includes an endpoint agent 616.

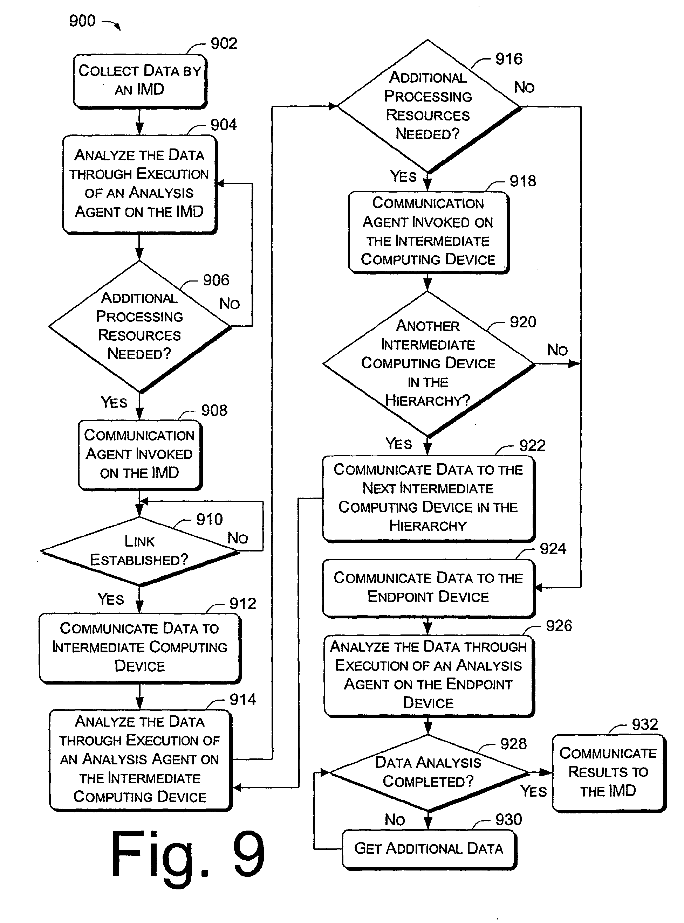

The execution of the agents 610-616 may be utilized to manage data in the hierarchy 600. The IMD agent 610, for example, may determine that data collected by the IMD 102 may require additional processing that is not available to the IMD agent 610, itself. Therefore the IMD agent 610, when executed, may communicate the data to a next tier of the hierarchy 600, i.e. the second tier 604, having additional processing resources. Similar determinations may be made through execution of the patient wearable unit and programmer agents 612, 614. Thus, data collected by the IMD 102 may be communicated through successive tiers 602-608 of the hierarchy 600 to utilize additional processing capabilities that are available in the hierarchy 600. An additional example of agent execution according to processing capabilities may be found in relation to Fig. 9. Similar determinations may be made through execution of the agents 610-616 based on memory capabilities, and so on. For example, each of the agents may be executed to migrate data through successive tiers of the hierarchy 600 when additional memory resources are required on the respective tiers, further discussion of which may be found in relation to Figs. 10 and 11.

Additionally, the agents 610-616 may manage data in the hierarchy 600 based on data criticality in the operation of the respective tiers 602-608. For example, each successive tier 604-608 of the hierarchy 600 of Fig. 6 after the first tier 602 is "further away" when communicating with the IMD 102. For instance, the hierarchy 600 may be configured such that communication is provided between successive tiers 602-604-606-608 of the hierarchy 600. Therefore, data may be stored at different tiers 602-608 of the hierarchy based on the data's criticality to operation of the IMD 102, such as to provide treatment to a patient. For instance, data that is more likely to be used in the operation of the IMD 102 may be stored in tier 604, while data that is less likely to be used by the IMD 102 may be stored in tier 606.

Fig. 7 is a block diagram showing an agent hierarchy 700 having exemplary agents for inclusion on one or more of the computing devices of the agent hierarchy 700. The agents 610-616 on each of the respective computing devices of the hierarchy 700 provide intelligent decision-making capabilities to perform defined tasks. The agent hierarchy 700 depicted in Fig. 7 arranges the agents 610-616 according to whether the agent is internal to the patent (e.g., agent 610) or external to the patient (e.g., agents 612-616). The agents may be configured to perform a variety of tasks, such as management of critical events (e.g., recording, processing, notification, etc.), memory resources, processing resources, archiving, software upgrades, device reprogramming (e.g., readjustment of parameters used by a patent diagnostic algorithm to analyze the patient, replace of patient diagnostic algorithms, etc.), patient data from a plurality of sources, process automation for data and communication processes, and so on. Each of these tasks is described in the following discussion as being performed by one or more agents that are configured to address the specific tasks. However, each of the agents may also be combined with another one or more of the agents as desired as illustrated in Fig. 6.

The agent hierarchy 700 shows IMD agent 610 as associated with the IMD 102 that is internal to the patient. The PWU agent 612, the PRO agent 614, and the ED agent 616 are associated with respective external devices, such as the intermediate computing devices 202(1), 202(m) and the endpoint device 140. As previously described, each of the agents 610-616 may be configured to address the differing capabilities of respective devices when implemented internally within the patient or external from the patient. The IMD agent 610, for instance, which is illustrated as being executed on the IMD 102 may include a plurality of agents, such as a critical events agent 702, one or more communication agents 704, one or more work-flow automation agents 706, and so on. The critical events agent 702 is executed on the IMD 102 to monitor, capture and process data collected by the IMD 102. For example, the critical events agent 702 may analyze the data collected by the IMD 102 to determine if the patient is experiencing, has experienced, and/or will experience a critical event relating to the health of the patient. The critical events agent 702 may then invoke the communication agent 704 to communicate data describing the critical event through the agent hierarchy 700 to a knowledge worker and/or to the patient.

The communication agent 704 is executed to manage communications between the IMD 102 and other computing devices of the agent hierarchy 700. For example, the communication agent 704 may include a network management agent that, when executed by the IMD 102, locates and identifies other computing devices that are communicatively coupled to the IMD 102. The communication agent 704 may also be executed such that the IMD 102 may communicate with the other computing device. For instance, the communication agent 704 may also include an interoperability agent that manages communication protocols and interoperability of the IMD 102 with other computing devices. The interoperability agent determines which one of a plurality of wireless protocols are to be used to communicate with another computing device, data types supported by the other computing device, and so on.

The IMD 102 may also include one or more work-flow automation agents 706. The work-flow automation agents 706, when executed, provide decision support which may be utilized to notify the patient and/or knowledge worker of the patient's condition. For example, the work-flow automation agents may utilize one or more diagnostic algorithms that analyze data collected by the IMD 102. Data resulting from this analysis may then be communicated through the hierarchy to the endpoint device 140 to provide context-sensitive information that may be utilized by the knowledge worker to help diagnose the patient's condition, monitor operational parameters of the IMD 102, and so on.

External computing devices of the agent hierarchy 700 may also include a critical events agent 708, one or more communication agents 710 and one or more work-flow automation agents 712 that correspond, respectively, to the critical events agent 702, communication agent 704, and work-flow automation agent 706 of the IMD 102. Each successive agent included on each respective computing device of the hierarchy 700, however, may have additional capabilities that are not available to a previous agent in the agent hierarchy 700. For example, each critical events agent may utilize additional patient diagnostic algorithms that are not available to a previous critical events agent in the agent hierarchy 700. In another example, each critical events agent may utilize diagnostic algorithms that require additional computational resources, such as additional processing and/or memory resources. A critical events agent, for instance, may execute a diagnostic algorithm that analyzes data stored on the intermediate computing device 202(m), e.g. a programmer. The intermediate computing device 202(m) may also store patient data for a predetermined amount of time, such as a week. The critical events agent executed on the endpoint device 140, however, may have access to all previous data that was collected by the IMD 102. Therefore, the critical events agent executed on the endpoint device 140 may have additional capabilities that are not available to the critical events agent executed on the intermediate computing device 202(m).

The external computing devices are illustrated in Fig. 7 as each including a data management agent 710. The data management agent 710, when executed, may provide a variety of functionality. For example, the data management agent 710 may check data integrity and memory constraints of each respective computing device. The data management agent 710 may also control distribution of data between computing devices in the hierarchy 700, such as to determine available memory resources and provide for distributed backup of data, e.g. archiving the data. The data management agent 710 may also be utilized to search, filter and analyze data, such as to determine which data is to be stored on the respective computing device based on criticality of the data. The data management agent 710 may also provide access and transactional security, such as to control device programming and software upgrades. In the agent hierarchy 700 of Fig. 7, the data management agent 710 is not included on the IMD 102 to reduce the amount of computational resources needed by the IMD 102. In another example, however, the IMD 102 may include a data management agent.

The critical events agent 702, 708, data management agent 714, and work-flow automation agent 706, 712 were each described as having one or more capabilities to analyze data collected by the IMD 102. Thus, in additional implementations the analysis functionality provided by these agents may be referred to collectively as being provided by an analysis agent.

### Operation of the Agent Hierarchy

The following discussion describes a variety of processes that may be implemented utilizing the previously described agent hierarchy. Aspects of each process may be implemented in hardware, firmware, or software, or a combination thereof.

Fig. 8 shows a process 800 for managing data in a network system based on analytical capabilities of one or more computing devices included in the network system. The process 800 is shown as a set of blocks that specify operations performed by one or more devices. At block 802, data is collected by the IMD. The IMD, for example, may collect patient data that describes the patient's condition. The IMD may also collect data that describes operation of the IMD itself, such as operational data describing battery power, available memory capabilities, software being executed on the IMD, and so forth.

At block 804, distribution of the data from block 802 is managed on a plurality of computing devices that implement the agent hierarchy. At block 806, the data is analyzed utilizing analytical capabilities of an agent that is executed on one of the computing devices. For example, the IMD may execute an agent that analyzes the data by utilizing an algorithm having one or more parameters that relate to expected ranges of the data. The agent may compare data with expected ranges of corresponding data to determine the patient's condition.

At decision block 808, a determination is made based on the analysis of block 806 to determine whether to notify one or more designated users of the patient's condition. For example, the analysis at block 806 may indicate that the patient's condition is worsening. Therefore, at block 810 designated users may be notified of the patient's condition, such as one or more knowledge workers and/or the patient. A notification may be sent to a knowledge worker in a variety of ways, such as via a telephone, fax, desktop computer and/or laptop computer as illustrated. The notification may also be provided to the intermediate computing device 202(m) configured as a patient wearable device to notify the patient.

If a result of the decision at block 808 is that notification is not needed, then at decision block 812 a determination is made at to whether the data is to be communicated to another computing device having additional analytical capabilities. For example, the other computing device may have analytical capabilities that are not included on the computing device that performed the analysis at block 806. If additional analytical capabilities are not needed, the computing device returns to block 806 to analyze additional data. The determination may be performed in a variety of ways. In one example, the agent executes a diagnostic algorithm that returns a result indicating whether further analysis is desired. In another example, the agent determines that even though the agent may analyze the data according to a desired algorithm, that such analysis could be more quickly performed by another agent executed on another computing device having additional hardware and/or software capabilities.

If additional analytical capabilities are needed, then the data is communicated to the computing device having the additional analytical capabilities, such as an intermediate computing device (block 814). Blocks 806, 808, 812, 814 of the process 800 may then be repeated by successive computing devices in the hierarchy. Thus, in this process 800, data collected by the IMD at block 802 is communicated through the hierarchy using successive computing devices when additional analytical capabilities are desired for analyzing the patient data.

Fig. 9 shows a process 900 for managing data in an agent hierarchy based on processing capabilities of one or more computing devices included in the agent hierarchy. At block 902, data is collected by an IMD. As previously described, the data may relate to the patient's condition and/or operation of the IMD itself. At block 904, the data is analyzed by the IMD through execution of an analysis agent on the IMD, such as to determine the patient's condition. At decision block 906, a determination is made to decide whether additional processing resources are needed to analyze the data. If not, the process 900 returns to block 904.

If additional processing resources are needed, the analysis agent on the IMD invokes the communication agent on the IMD to communicate the data to another computing device at block 908. At decision block 910, the communication agent determines whether a link is established with an intermediate computing device. Once established, the data is communicated to the intermediate computing device (block 912).

At block 914, the intermediate computing device executes an analysis agent. The analysis agent, when executed, analyzes the data and decides whether additional processing resources are needed to further analyze the data (block 916). For example, the intermediate computing device may be configured as a patient wearable device that, while having processing resources that are greater than the IMD, may still have limited processing resources due to size and/or battery limitations of the patient wearable device. Therefore, the agent on the intermediate computing device may execute one or more patient diagnostic algorithms that analyze the data to monitor the patient's condition which are not available to the agent on the IMD. These algorithms, when executed, may also be utilized to determine whether additional processing is needed. For instance, the data, when analyzed through execution of the agent, may indicate that additional patient diagnostic algorithms should be utilized to analyze the data.

At block 918, the analysis agent executed on the intermediate computing device may then invoke a communication agent if additional processing resources are needed. At block 920, the communication agent, when executed, may then determine whether an additional intermediate computing device is available in the hierarchy. If so, the communication agent communicates the data to the next intermediate computing device in the hierarchy at block 922. The next intermediate computing device may then repeat blocks 914-922 of the process 900 to analyze the data and determine whether additional processing resources are needed. In this way, the data may be communicated through successive tiers of the hierarchy for analysis by agents that are executed on computing devices having additional processing resources.

At decision block 920, if another intermediate computing device is not available in the hierarchy, then the data is communicated to the endpoint device through execution of the communication agent (block 924). At block 926, the endpoint device also analyzes the data through execution of an analysis agent to determine the patient's condition as previously described. At block 928, the analysis agent, when executed on the endpoint device, may also determine whether analysis may be completed from data available to the endpoint device. For example, analysis of the data by the analysis agent may indicate that a particular patient diagnostic algorithm should be executed to analyze the data. The patient diagnostic algorithm, however, may require additional data that is not currently available on the endpoint device, such as additional data utilized to indicate a trend over a specified period of time. Therefore, at block 930, the communication agent is executed to request additional data. The requested data may be obtained from a variety of computing devices in the hierarchy. For example, the communication agent may communicate with the IMD, either directly or through the intermediate computing devices, to request additional data from the IMD. Additionally, the communication agent may request data that is stored on one or more of the intermediate computing devices, such as processing results of one or more patient diagnostic algorithms on the intermediate computing devices that are not available on the endpoint device. Thus, the hierarchy may support two-way communication. For example, data describing a patient's condition may flow "up" from the IMD through the intermediate computing devices to the endpoint device, and the endpoint device may also communicate back "down" through the hierarchy to obtain data from the intermediate computing devices and/or the IMD. At block 932, the results of the analysis of the data are communicated to the IMD.

Fig. 10 shows a process 1000 for managing data in an agent hierarchy based on data criticality and memory capabilities of one or more computing devices included in the agent hierarchy. At block 1002, the IMD executes an agent to examine memory resources on the IMD. For example, the agent may determine a percentage of the memory resources on the IMD that are available to store data collected by the IMD that describes the patient's condition. The agent may also intelligently determine an amount of memory resources that are available based on amount of data that is collected by the IMD. For instance, the agent may establish that the IMD collects a particular amount of data during a particular amount of time to determine the amount of memory resources that are available on the IMD.

At decision block 1004, the agent determines if additional memory resources are needed to store the data collected by the IMD. The agent, for instance, may compare the amount of memory resources available from the examination of block 1002 with a threshold amount of memory resources. If additional memory resources are needed, the agent communicates at least a portion of the data to an intermediate computing device at block 1006. In this instance, the agent employs a first-in/first-out (FIFO) mechanism to communicate the oldest data. The agent may also employ a variety of other mechanisms.

At decision block 1008, the intermediate computing device, like the IMD, determines if additional memory resources are needed on the intermediate computing device. If not, the process 1000 returns to block 1002. If additional memory resources are needed, the intermediate computing devices execute an agent to examine criticality of the data and previously stored data on the intermediate computing device (block 1010). For example, the intermediate computing device may include data that was previously collected on the IMD and communicated from the EMD to the intermediate computing device. The agent, when executed, may then examine the previously stored data as well as the communicated data to determine which data is most critical to operation of the network system.

Data criticality may be based on a variety of factors. For instance, the data may describe times at which the IMD administered therapy to the patient, such as to restore a normal heart rhythm. Such data may remain stored on the intermediate computing device as well as communicated "up" the hierarchy to additional computing devices, e.g. the endpoint device, for further analysis. Therefore, the intermediate computing device may save data that may be relevant to future diagnosis of the patient and also communicate that data for further analysis.

In another instance, the network system may implement an archiving hierarchy to archive older and greater amounts of data on respective successive tiers of the hierarchy. The criticality of the data, in this instance, may therefore reflect the archiving hierarchy such that newer data is stored "closer" to the IMD for faster communication with the IMD. For example, an intermediate computing device that is directly communicatively coupled with the IMD may have additional analytical capabilities as well as additional memory capabilities to store a greater amount of the patient's diagnostic data than the IMD. The intermediate computing device may analyze the data and determine from the analysis that the IMD should perform one or more therapeutic operations. The intermediate computing device may therefore communicate directly with the IMD and thus initiate the therapeutic operation in a quicker manner than if the intermediate computing device had to communicate with the IMD through one or more additional computing devices.

At decision block 1012, the intermediate computing device, through execution of the agent, determines if an additional computing device is available. The agent, for instance, may include a communications agent that, when executed, determines if other computing devices are communicatively coupled to the intermediate computing device over a network. If an additional computing device is not available, then at block 1014, the data is stored until the memory is full, after which, the least critical data is replaced in the memory with data having a higher criticality as was described in relation to block 1010. If an additional computing device is available, the least critical data is communicated to the next computing device at block 1016 and the process 1000 continues at block 1008. Thus, in this exemplary process 1000, data is communicated "up" successive tiers of the hierarchy based on memory resources and criticality of the data. Data may be managed in a variety of other ways in the network system, additional examples of which are described in relation to Fig. 11.

Fig. 11 shows a hierarchy 1100 implemented in a network system in which data is managed through execution of a plurality of agents by respective computing devices. The illustrated network system includes a plurality of computing devices, such as the IMD 102, intermediate computing device 202(1)-202(M), and the endpoint device 140 that were previously described in relation to Fig. 2. Each of the computing devices includes a respective one of a plurality of agents 1102-1110 that, when executed, manage respective memory 1112-1110 resources on the computing devices for storing data, such as the data collected by the IMD 102. In Fig. 11, memory 1112-1120 included on each respective computing device is configured to store greater amount of data. This is represented pictorially in Fig. 11 as data 1122(1)-1122(2) stored on memory 1112, data 1124(1)-1124(3) stored on memory 1114, data 1126(1)-1126(4) stored on memory 1116, and data 1128(1)-1128(5) stored on memory 1118. In this implementation, memory 1120 included on the endpoint device 140 may be considered virtually limitless in that the memory 1120 may be configured as one or more memory devices that store vast amount of data, such as a RAID array. In other words, memory 1120 may act as a central repository to store all the data collected by the IMD.

The agents 1102-1110, when executed on respective computing devices, manage data in the hierarchy in a variety of ways. For example, as previously described in relation to Fig. 10, each of the plurality of agents 1102-1108 may monitor respective memory 1112-1118. If additional memory resources are needed, the agent may communicate the data to a successive computing device in the hierarchy 1100. Thus, in this example the agents 1102-1108 monitor respective memories 1112-1118 without being "aware" of the status of other memories in the hierarchy 1110 and therefore "push" the data to the next tier of the hierarchy.

In another example, the agents 1104-1110 may "pull" data from a previous tier in the hierarchy. For instance, agent 1104 may be executed on the intermediate computing device 202(1) to monitor the memory 1112 of the IMD 102. The agent 1104 may monitor the memory in a variety of ways, such as through direct monitoring by the agent 1104 or through communication with the agent 1102 that is executed on the IMD 102. When the memory 1112 passes a described threshold, the agent 1104 may read and erase data from the memory 1112 to make additional memory resources available to the agent 1102 on the IMD 102. Thus, in this example, each of the agents 1104-1110 is executable to examine data located on another computing device in the hierarchy 1100.

In yet another example, each of the agents 1102-1110 of the hierarchy may communicate, one to another, to manage data in the network system as a whole. Each of the agents 1102-1110, for instance, may communication with other agents 1102-1110 in the hierarchy 1100 to determine the memory resources on each computing device in the hierarchy, amount of data stored by each of the computing devices, and so on. Communication may be performed in a variety of ways. For instance, each agent may communicate with agents located immediately "next" to the agent. For example, agent 1104 may communicate directly with agent 1102 and agent 1106 to discover available memory resources on the IMD 102 and the intermediate computing device 202(m), respectively. The agent 1104 may also communicate data regarding the memory resources of the intermediate computing device 202(m) to the IMD 102. Thus, the IMD 102 may be made "aware" of both the presence and the memory resources of the intermediate computing device 202(m). In another implementation, each of the agents 1102-1110 may communicate directly with other agents of the hierarchy 1100.

Further, communication between agents 1102-1110 in the hierarchy 1100 may be utilized for dynamic management of data in the hierarchy. For instance, the agents 1102-1110 may respond to changing states of each of the computing devices in the hierarchy and rearrange the data according. In an example, the data is communicated based on pending unavailability of one or more of the computing devices, which may be caused by a hardware, software, and/or network failure. Intermediate computing device 202(m) executes the agent 1106, for instance, which determines that failure of the intermediate computing device 202(m) is imminent, such as through one or more diagnostic algorithms that monitor the operation of the computing device. In another instance, the intermediate computing device 202(m) may become unavailable due to removal of the device from the network system, such as when a patient leaves a doctor's office. The agent 1106, in response to the impending unavailability, communicates data 1126(1)-1126(2) to intermediate computing device 202(1) and data 1126(3)-1126(4) to intermediate computing device 202(M). Thus, the data may remain available to other agents 1102, 1104, 1108, 1110 of the hierarchy 1100 even when the intermediate computing device 202(m) is no longer available.

One or more of the agents 1102-1110 may also be executed to provide a variety of other data management mechanisms. For instance, agent 1102 may be executed on the IMD 102 at periodic intervals to upload data from the IMD 102 through the intermediate computing devices 202(1)-202(M) to the endpoint device 140. In another instance, the agents 1102-1110 may be executed to communicate software upgrades from the endpoint device 140 to the intermediated computing devices 202(1)-202(M) and the IMD 102 as described in relation to Fig. 13. A variety of other data management mechanisms may also be employed to manage patient and other data in the network system.

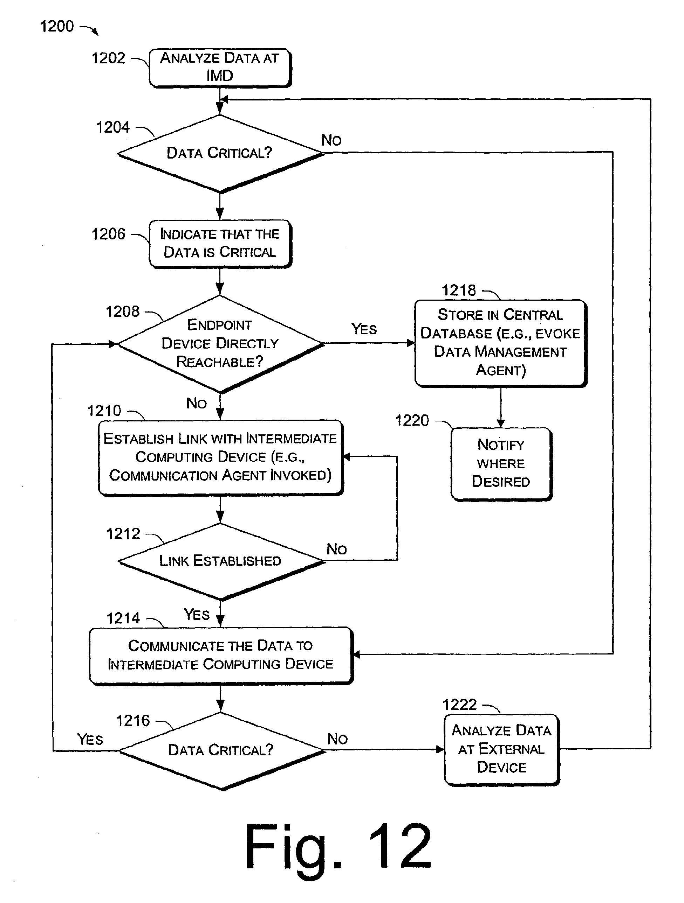

Fig. 12 shows a process 1200 for managing critical data in a network system. To quickly communicate critical data through the network system, data may be indicated as critical such that the data is communicated before analysis is performed for determining the patient's condition by the intermediate computing devices. For example, at block 1202, data is analyzed by an agent utilizing one or more patient diagnostic algorithms. At decision block 1204, a determination is made to decide whether the data is critical. If so, the data is indicated as critical at block 1206. The indication may be provided in a variety of ways, such as by setting a flag, addition of one or more parameters to the data which describe the criticality of the data, and/or inclusion of the results of the analysis performed at block 1202.

At decision block 1208, a determination is made to determine whether the endpoint device is directly reachable. For example, a communication agent may be executed to locate the endpoint device and determine whether the device is reachable over a network. For instance, the IMD may perform the analyzing, determining, and indicating of blocks 1202-1206. The IMD, however, may not have sufficient power to directly communicate with the endpoint device. At block 1210, the IMD may communicate the data to an intermediate computing device by establishing a link with an intermediate computing device by invoking a communication agent. If a link is established at block 1212, then the data is communicated to the intermediate computing device (block 1214).

At decision block 1216, the intermediate computing device may determine whether the data is critical. For example, the indication provided at block 1206 may cause the intermediate computing device to automatically invoke a communication agent to immediately communicate the data. Thus, the data may be communicated immediately by the intermediate computing device without being first analyzed using one or more patient diagnostic algorithms. If the data is critical, the process 1200 returns to block 1208 such that the intermediate computing device determines if the endpoint device is directly reachable. Continuing with the previous example, the intermediate computing device, for instance, may be configured as the networked programmer 114 of Fig. 1 which is communicatively coupled to the endpoint device 140 over the network 118. Therefore, the intermediate computing device may then communicate the data directly to the endpoint device 140.

At block 1208, if the endpoint is directly reachable, then the critical data is communicated to the endpoint device, which then stores the data in a central database (block 1218). At block 1220, the endpoint device also determines whether to notify a knowledge worker to the existence of the critical data. For instance, if the knowledge worker is carrying a PDA or phone, a statement may be sent by the endpoint device that "Patient X has an irregular heart beat".

If the data is not determined to be critical at block 1204, the process 1200 moves to block 1214 to communicate the data to an intermediate computing device. If a result of the determination at block 1216 is that the data is not critical (e.g., a critical flag is not set in the data), then the data is analyzed at block 1222 by the intermediate computing device using one or more patient diagnostic algorithms and the process returns to block 1204. In this way, each successive tier of the hierarchy may determine whether data is critical using one or more patient diagnostic algorithms. When the data is determined to be critical, the data is communicated to the endpoint device without first being analyzed by other intermediate computing devices. Even though the data is communicated without first being analyzed, however, the data may still be analyzed by each successive tier through execution of respective analysis agents.

Fig. 13 shows a hierarchy 1300 implemented in a network system in which data is communicated from the endpoint device through tiers of the hierarchy to the IMD through execution of a plurality of agents by respective computing devices. As previously discussed in relation to Figs. 9 and 11, data may be communicated both from the endpoint device 140 to the IMD 102 through the intermediate computing devices 202(1)-202(M) and vice versa. Two-way communication may be utilized to provide a variety of functionality.

In one example, software updates 1302(1)-1302(4) (updates) are communicated from the endpoint device 140 to respective computing devices of the hierarchy 1300. For instance, the endpoint device 140 may specify updates that are for specific computing devices of the hierarchy 1300. Updates 1302(1) may be configured for the intermediate computing device 202(M) and therefore communicated directly from the endpoint device 140 to the intermediate computing device 202(M) over the network 118. Updates 1302(4), however, are for updating software on the IMD 102, such as the agent 1102. Therefore, the updates 1302(4) are communicated from the endpoint device 140 through each of the intermediate computing devices 202(1)-202(M) to the IMD 102. Similar techniques may be utilized to communicate updates 1302(2), 1302(3) to intermediate computing devices 202(m), 202(1), respectively.

Likewise, parameters 1304(1)-1304(4) may be communicated from the endpoint device 140 to the intermediate computing devices 202(1)-202(M) and/or the IMD 102. The parameters 1304(1), 1304(2), 1304(3), 1304(4) may be utilized by respective agents 1108, 1106, 1104, 1102 in analyzing the patient's condition as was previously described in relation to Fig. 4. For instance, parameters 1304(3) may be utilized to readjust parameters employed by one or more patient diagnostic algorithms of agent 1104. Thus, the parameters 1304(3) may be utilized to readjust the analysis performed through execution of the agent 1104. In another instance, parameters 1304(4) are utilized by the agent 1102 of the IMD 102 to control therapeutic operations performed by the IMD 102. For example, the parameters 1304(4) may specify changes to be made in the operation of the IMD 102, such as to provide cardiac stimulation. Thus, the endpoint device 140 may readjust therapy provided by the IMD 102 through communication of the parameters 1304(4).

Fig. 14 shows a hierarchy 1400 implemented in a network system in which data is communicated from a plurality of IMDs and external medical devices through tiers of the hierarchy to the endpoint device through execution of a plurality of agents by respective computing devices. In the previous description, data was described as being collected by a single IMD 102 which was represented pictorially as an ICSD. The hierarchy 1400, however, may also include a plurality of the IMDs as well as external medical devices that provide additional data that is utilized to describe a patient's condition.

IMD 1402, for instance, may be configured as an implantable medical dosing device that outputs one or more medications internally to the patient 104. The patient 104 may also include an external medical device 1404 (EMD) that is configured to collect patient diagnostic data externally from the patient 104, such as a blood-pressure monitor. Data from the plurality of medical devices, i.e. IMD 102, IMD 1402, EMD 1404, may be aggregated for analysis by an analysis agent 1406 executed on an intermediate computing device 202(m). For example, the analysis agent 1406 may include an aggregating agent 1408 that combines data points from the various medical devices. The aggregating agent 1408 may intelligently combine the data points such that similar data points are compared for inconsistencies and omissions. For example, the IMD 102 and the EMD 1404 may both provide patient diagnostic data that describes a patient's pulse. The aggregating agent 1408, when comparing the data from both devices, may therefore check the operation of the devices. Additionally, data provided by one of the devices may be utilized as a backup should one of the other devices fail.

The analysis agent 1406, when executed, may then utilize the data from the variety of devise to analyze the patient's condition. The combined data may also be communicated to the endpoint device 140 for analysis by an analysis agent 1410 executed thereon as previously described.

## Claims

1. A system comprising: at least one implantable medical device (IMD) that collects data; and a plurality of computing devices that are communicatively coupled, one to another, **characterized in that**: each of the computing devices has capabilities to analyze the data; at least one of the computing devices is communicatively coupled to the IMD to obtain the data; and one or more of the computing devices is configured to determine whether to communicate the data to another one of the computing devices having analytical capabilities that are not included in the analytical capabilities of the one or more computing devices.

2. A system as claimed in Claim 1, **characterized in that** one or more of the computing devices is further configured to form a communication for notifying a designated user of a result of the analysis.

3. A system as claimed in Claim 1 or Claim 2, **characterized in that that** the analytical capabilities are selected from processing capabilities, memory resources, network resources, patient diagnostic algorithms, device diagnostic algorithms and any combination thereof.

4. A system as claimed in any preceding claim, **characterized in that** the IMD is configured to determine whether to communicate the data to one of the computing devices that has analytical capabilities in addition to the analytical capabilities of the IMD.

5. A system as claimed in any of Claims 1 to 3, **characterized in that** the IMD is configured as a device selected from a cardiac stimulation device, a cardiac monitor, a medicine dosing device, a neurological device for at least one of monitoring and stimulating nerves and any combination thereof.

6. A system as claimed in any preceding claim, **characterized in that** the computing network is configured to distribute the data to computing devices selected from a group of computing devices including a computer, a portable computer, a personal digital assistant, a wireless phone, a facsimile, and a database.

7. A system comprising: a hierarchy of computing devices that are communicatively coupled, one to another, **characterized in that**: at least one of the computing devices is an implantable medical device (IMD); the hierarchy is defined by each respective amount of data that each of the computing devices is configured to store; and one or more of the computing devices is configured to determine whether to communicate the data to another one of the computing devices that is configured to store a different amount of data.

8. A system as claimed in Claim 7, **characterized in that** the said one or more computing devices make the determination based on how much data is stored in the memory of the respective system.

9. A system as claimed in Claim 7 or Claim 8, **characterized in that** at least two of the computing devices are IMDs; and one or more of the computing devices is configured to aggregate data collected by the IMDs.

10. A system as claimed in any of claims 7 to 9, **characterized in that** at least one other computing device is an external medical device (EMD); and one or more of the computing devices is configured to aggregate data collected by the IMD and the EMD.

11. A system as claimed in any of Claims 7 to 10, **characterized in that** the said one or more computing devices are further configured to: analyze the data; and form a communication, based on the analysis, for notifying a designated user of the analysis.
